# FASCICULE DE BREVET EUROPEEN

(11) **EP 0 838 681 B1**
(45) Date de publication et mention de la délivrance du brevet: **12.01.2005**
(21) Numéro de dépôt: 97402117.2
(22) Date de dépôt: 12.09.1997
(51) Int. Cl.: G01N 33/80, G01N 33/50

(54) **Procédé d'évaluation de la phagocytose des érythrocytes par des macrophages in vitro ; application au contrôle de qualité de stocks d'érythrocytes.**
Verfahren zur Auswertung der Makrophagen-Phagozytose von Erythrozyten; Verwendung in der Qualitätsprüfung von gelagerten Erythrozyten
Method to evaluate the phagocytosis of erythrocytes by macrophages; use in quality control of stored erythrocytes

(30) Priorité: 16.09.1996 FR 9611250
(43) Date de publication de la demande: 29.04.1998
(73) Titulaire: ASSOCIATION POUR L'ESSOR DE LA TRANSFUSION SANGUINE DANS LA REGION DU NORD, 59000 Lille (FR); UNIVERSITE DES SCIENCES ET TECHNOLOGIES DE LILLE, 59655 Villeneuve d'Ascq (FR)
(72) Inventeur: Bratosin, Daniela, 59650 Villeneuve D'Ascq (FR); Mazurier, Joel, 59650 Villeneuve D'Ascq (FR); Montreuil, Jean, 59650 Villeneuve D'Ascq (FR); Huart, Jean-Jacques, 59000 Lille (FR)
(74) Mandataire: Lhuillier, René

(56) Documents cités:
- EP-A- 0 435 226
- WO-A-96/14737
- BRATOSIN ET AL.: "Flow cytofluorimetric analysis of young and senescent human erythrocytes probed with lectins. Evidence that sialic acids control their life span" GLYCOCONJUGATE JOURNAL, vol. 12, no. 3, juin 1995, pages 258-267, XP000615968
- VAN AMERSFOORT ET AL.: "Evaluation of a flow cytometric fluorescence quenching assay of phagocytosis of sensitized sheep erythrocytes by polymorphonuclear leukocytes." CYTOMETRY, vol. 17, no. 4, 1 décembre 1994, pages 294-301, XP000674142
- LEB ET AL.: "Antiglobulin serum mediated phagocytosis of normal senescent and oxidized RBC: role of anti-IgM immunoglobulins in phagocytic recognition." BRITISH JOURNAL OF HAEMATOLOGY, vol. 66, no. 4, août 1987, OXFORD, pages 565-570, XP000197537
- VADDI ET AL.: "Modulation of Fc tau receptor expression and function in mouse peritoneal macrophages by ammonium metavanadate." INTERNATIONAL JOURNAL OF IMMUNOPHARMACOLOGY, vol. 13, no. 6, 1991, OXFORD, pages 1167-1176, XP000197546
- DATABASE WPI Section Ch, Week 9519 Derwent Publications Ltd., London, GB; Class B04, AN 95-145677 XP002031710 & SU 1 837 231 A (PERM MED INST) , 30 août 1993
- SHINOZUKA: "Changes in human red blood cells during aging in vivo." THE KEIO JOURNAL OF MEDICINE, vol. 43, no. 3, septembre 1994, TOKYO, pages 155-163, XP000197538

## Description

L'invention concerne un procédé d'évaluation de l'état physiologique des érythrocytes, par la quantification de la phagocytose desdits érythrocytes, *in vitro,* par des macrophages en culture. Le procédé comprend un marquage fluorescent des érythrocytes, leur phagocytose éventuelle par les macrophages et la détermination du marquage des macrophages résultant de cette phagocytose par cytofluorimétrie en flux.
L'intérêt de cette évaluation provient de la corrélation entre l'importance de la phagocytose des érythrocytes et leur état physiologique lié à des changements de constituants membranaires. Elle permet ainsi d'évaluer le degré de sénescence des érythrocytes, en particulier destinés aux transfusions sanguines.

Il est connu que les érythrocytes ont une durée de vie de 120 jours, dans la circulation sanguine. Ensuite ils sont phagocytés par les macrophages. Le mécanisme de l'érythrophagocytose est lié à la perte de molécules d'acide sialique des glycoprotéines et des glycolipides des membranes des érythrocytes sénescents. (Bratosin et al. Glycoconjugate J. 12, 1995, 258-267).

Les inventeurs ont émis l'hypothèse et ont vérifié que le vieillissement des érythrocytes au cours de leur stockage dans les centres de transfusion induit un phénomène de désialylation analogue à celui qui se produit au cours de la sénescence *in vivo,* ce qui permet d'expliquer que 3 jours après une transfusion, on ne retrouve que 30 % d'érythrocytes fonctionnels.

Le rôle central de la phagocytose par les macrophages, dans les phénomènes observés, a conduit la Demanderesse à mettre au point un procédé simplifié permettant de quantifier cette phagocytose, de manière simple, rapide, sensible et reproductible, pour en tirer diverses applications à but thérapeutique ou diagnostique.

Ainsi la Demanderesse a mis en évidence la capacité de macrophages mis en culture *in vitro* de remplir leur fonction naturelle de phagocytose de cellules étrangères ou anormales, comme les érythrocytes sénescents. La Demanderesse en a déduit un procédé permettant une mesure objective de cette capacité de phagocytose, par le marquage artificiel des érythrocytes par un fluorochrome, leur phagocytose in vitro et la mesure du marquage consécutif des macrophages ayant phagocyté les érythrocytes, par cytofluorimétrie en flux.

Le procédé selon l'invention comprend la succession des étapes suivantes :
a) la mise en culture de macrophages ;
b) le marquage des membranes des érythrocytes par un fluorochrome non cytotoxique ;
c) la mise en contact des érythrocytes marqués avec la culture de macrophages pendant le temps nécessaire pour permettre leur phagocytose ;
d) l'élimination par lavage des érythrocytes non phagocytés et la lyse des érythrocytes adhérents mais non phagocytés ;
e) l'élimination du fluorochrome des membranes résiduelles desdits érythrocytes adhérents par un traitement à l'éthanol à 70 % ;
f) la récolte des macrophages ;
g) la mesure et l'enregistrement du marquage fluorescent des macrophages.

Pour l'étape a., on utilise de préférence des macrophages péritonéaux de souris fraîchement prélevés, qui sont incubés en conditions de culture standard à 37°C, pendant environ une heure en absence de sérum de veau foetal puis pendant 2 à 6 heures en présence dudit sérum.

Pour l'étape b., le fluorochrome sera plus particulièrement choisi parmi les colorants lipophiles non cytotoxiques, se fixant spécifiquement et irréversiblement dans les membranes cellulaires, comme le colorant connu sous l'appellation PKH-26 (produit par Zynaxis Cell Science Inc. USA et vendu par Sigma).
Ce fluorochrome est constitué par fixation covalente d'une chaîne aliphatique à 26 carbones sur l'acridine orange. Cette "queue" hydrophobe fixe en quelques minutes le marqueur fluorescent dans la couche lipidique de la membrane cellulaire, de manière irréversible et sans en modifier l'intégrité.
Tout autre fluorochrome qui présenterait les mêmes avantages pourrait également être utilisé.

Pour l'étape c., les érythrocytes marqués seront mis en contact avec la culture de macrophages pendant 1 à 6 heures à 37°C.

Pour l'étape d., l'élimination des érythrocytes non phagocytés sera effectuée par 3 lavages en milieu physiologique, et complétée par la lyse des érythrocytes adhérant encore à la surface externe des macrophages par un traitement de 5 minutes en tampon hypotonique.

Pour l'étape e., la culture est traitée pendant 15 minutes avec une solution de lidocaïne, mise en suspension en tampon physiologique, et traitée à l'éthanol à 70 % pendant 20 minutes ce qui fixe les cellules et dissout la PKH-26 des fragments membranaires encore adhérents. Cette étape de traitement à l'éthanol est particulièrement avantageuse par sa simplicité de mise en oeuvre par rapport à d'autres procédés décrits comprenant un "quenching" différentiel par des fluorochromes sélectifs (Amersfoort et Van Strÿp - Cytometry 17 ; 1994 ; 294-301).

Pour l'étape f., les macrophages sont remis en suspension en milieu physiologique et sont récoltés.

Pour l'étape g., la mesure et l'enregistrement de la fluorescence des macrophages sont effectués de préférence au moyen d'un cytofluorimètre en flux, ladite mesure portant sur un nombre statistiquement significatif de macrophages, par exemple 5000 cellules pour chaque condition expérimentale. La mesure de la fluorescence est effectuée à deux longueurs d'onde, ce qui donne aux macrophages une autofluorescence verte et à la PKH-26 une fluorescence rouge. Les mesures enregistrées permettent ainsi de calculer, avec un logiciel approprié, le pourcentage de macrophages ayant phagocyté au moins une cellule et de visualiser, sur des histogrammes, deux régions, l'une (R1) de non-phagocytose et l'autre (R2) de phagocytose, ainsi que des sous-populations de macrophages présentant différents niveaux de phagocytose (R2a et R2b).

Le procédé selon l'invention est plus simple et plus rapide que les procédés utilisant la méthode de "quenching" ou le marquage radioactif au ⁵¹Cr et surtout, dans ce dernier cas, il évite une cause d'erreur liée aux variations aléatoires du comptage de la radioactivité et des macrophages.

Le premier domaine d'application du procédé selon l'invention est l'évaluation de la sénescence des érythrocytes au cours de leur stockage en vue des transfusions sanguines. Dans ce but, l'érythrophagocytose est mesurée au cours du temps et son augmentation est utilisée comme révélateur de modifications de la structure membranaire des érythrocytes, associées à leur sénescence.

Le procédé est particulièrement remarquable par sa simplicité et par sa rapidité, les résultats étant obtenus 8 heures après le prélèvement des macrophages péritonéaux, ce qui permet de l'utiliser comme test de routine.

Le même procédé permet également d'évaluer les améliorations des conditions de conservation des érythrocytes destinés aux transfusions sanguines.

Il peut aussi s'appliquer au contrôle de qualité et au suivi des améliorations des conditions de conservation des érythrocytes destinés aux analyses, notamment aux tests de compatibilité prétransfusionnels.

Le procédé trouve également des applications diagnostiques : par exemple, l'évaluation de l'état de sénescence des érythrocytes dans un prélèvement sanguin peut être utilisée pour identifier des états d'anémie qui nécessitent une transfusion sanguine (le pourcentage d'érythrocytes sénescents indiquant que le sujet ne régénère plus ou plus suffisamment de cellules immatures).

Un mode de mise en oeuvre du procédé selon l'invention est explicité dans l'exemple suivant, sans toutefois en limiter la portée.

Les résultats sont illustrés par les figures suivantes :
Figure 1 : Histogramme de cytofluorimétrie en flux de l'érythrophagocytose après 1 heure d'incubation des érythrocytes marqués à la PKH-26 et des macrophages péritonéaux.
   1 à 4 : nombre d'érythrocytes phagocytés.
   Abscisse : logarithme de l'intensité de la fluorescence.
   Ordonnée : nombre de cellules.
Figure 2 : Histogramme de cytofluorimétrie en flux de l'érythrophagocytose après 2 heures d'incubation (comme figure 1)
   - Pic 1 :: contrôle : inhibition de la phagocytose en présence d'azide de sodium
   - Pic 2 :: macrophages incubés en présence d'érythrocytes natifs
   - Pics 3A et 3B :: macrophages incubés en présence d'érythrocytes traités à la neuraminidase.
Figure 3 : Analyse graphique "dot-plot" de l'érythrophagocytose après 2 heures d'incubation
   - Ordonnée :: fluorescence rouge des érythrocytes marqués à la PKH-26.
   - Abscisse :: autofluorescence verte des macrophages.
   3A : contrôle en présence d'azide de sodium
   3B : macrophages incubés avec des érythrocytes natifs
   3C : macrophages incubés avec des érythrocytes traités à la neuraminidase
   R1 : région de référence des macrophages en absence de phagocytose
   R2 : région de référence de phagocytose.
   R2a et R2b : 2 sous-populations définies par l'intensité de leur fluorescence en rapport avec le nombre d'érythrocytes phagocytés.

### Exemple 1 - Evaluation de l'érythrophagocytose par des macrophages en culture.

### 1) Mise en culture des macrophages.

On utilise des souris (mâles et femelles) de souche Swiss âgées de 6 à 8 semaines. Les macrophages sont récoltés par lavage de la cavité péritonéale de souris avec 10 ml de solution physiologique de Hanks. La suspension de cellules est centrifugée à 350 g pendant 5 minutes à 4°C.

Le culot est resuspendu en milieu DMEM contenant 20 % de sérum foetal de veau. La suspension de cellules est inoculée sur boîtes de culture multipuits en polystyrène, à raison de 500 µl de suspension à 2-4 10⁵⁻⁶ cellules/ml par boîte à 24 puits de 16 mm de diamètre, et incubée pendant 4 heures à 37°C, à l'air humidifié et en présence de 5 % de CO₂.

Ensuite les cellules non adhérentes sont éliminées par 2 lavages en milieu DMEM et les macrophages adhérents sont utilisés pour les tests de phagocytose.

### 2) Marquage des érythrocytes.

Pour la mise au point du test et l'élaboration des courbes de référence, on utilise du sang humain collecté en présence d'héparine. Les érythrocytes sont séparés par centrifugation à 2000 g pendant 5 minutes à 4°C. Le culot est lavé 3 fois en tampon phosphate additionné d'un inhibiteur de protéase (PMSF, phénylméthylsulfonyl fluorure, 0,2 mM).

Le marquage avec le colorant fluorescent rouge PKH-26 est effectué selon les instructions du fournisseur (Sigma, St Louis, MO. USA - le colorant est fabriqué par Zynaxis - Malverne-PA-USA)

Brièvement, 50 µl de suspension d'érythrocytes (à 5 10⁸ cellules/ml) sont mis en suspension dans 1 ml du "diluent C" du kit et additionnés de 1 ml de PKH-26 2 µM. Le mélange est agité doucement pendant 4 minutes à 25°C. La réaction de marquage est arrêtée par l'addition de sérum humain, de milieu complet et par centrifugation des cellules à 2000 g pendant 5 minutes à 25°C. Le culot est lavé et prêt à l'emploi.

### 3) Desialylation des érythrocytes.

Pour obtenir un étalon de phagocytose maximale, un aliquot d'érythrocytes est désialylé par traitement à la neuraminidase de *Vibrio cholerae* (il est connu que les cellules d'origine humaine n'ont qu'un type d'acide sialique, l'acide N-acétylneuraminique et qu'il est très sensible à la neuraminidase) :

125 mU d'enzyme sont ajoutés à 1 ml de suspension d'érythrocytes (à hématocrite de 30 %) en tampon phosphate pH 7,4. Après 1 heure d'incubation à 37°C sous agitation, les érythrocytes sont centrifugés à 2000 g pendant 5 minutes à 4°C puis le culot est lavé 3 fois en tampon phosphate.

La désialylation doit être effectuée après le marquage au PKH-26, la désialylation préalable interdisant le marquage.

### 4) Mesure de la phagocytose.

Des aliquots de 2.10⁷ érythrocytes natifs ou traités à la neuraminidase, marqués au PKH-26, en suspension dans 250 µl de milieu DMEM sont mis en contact avec les cultures de macrophages et incubés dans 150 µl de sérum par puits, à 37°C, en air humidifié et à 5 % de CO₂. L'incubation est poursuivie pendant 1 heure pour obtenir une phagocytose limitée ou pendant 2 heures pour obtenir une phagocytose intense.

Les érythrocytes non absorbés sont éliminés par 3 lavages avec 0,5 ml de milieu DMEM.

Les érythrocytes adhérents aux membranes des macrophages mais non internalisés sont lysés par un traitement avec 0,5 ml de tampon hypotonique à pH 7,2 (NH₄Cl 140 mM, Tris 17 mM) suivi d'un lavage en tampon phosphate.

Les macrophages sont ensuite détachés de la boîte de culture par un traitement à la lidocaïne (4 mg/ml en tampon phosphate - EDTA). Après 15 minutes d'incubation sur de la glace (selon Rabinovitch et DeStefano - In vitro 11, 1975, 379), ils sont détachés à la pipette, récoltés, centrifugés à 350 g pendant 5 minutes à 4°C, puis lavés en tampon phosphate.

Ensuite les macrophages sont traités avec 70 % d'éthanol pendant 20 minutes à température ambiante ce qui fixe les cellules et dissout la PKH-26 des fragments membranaires des érythrocytes encore adhérents. Ensuite ils sont remis en suspension en tampon phosphate à pH 7,4.

La suspension de macrophages est ensuite analysée par cytofluorimétrie en flux à l'aide d'un appareil FAC Scan de Becton-Dickinson (San Jose, CA, USA), équipé d'un logiciel PC LYSYS pour l'analyse des résultats.

Pour chaque population, 5000 particules fluorescentes sont analysées.

On observe un "bruit de fond" de fluorescence endogène des macrophages natifs et des macrophages préincubés en présence de 1 % d'azide de sodium (puissant inhibiteur de phagocytose)

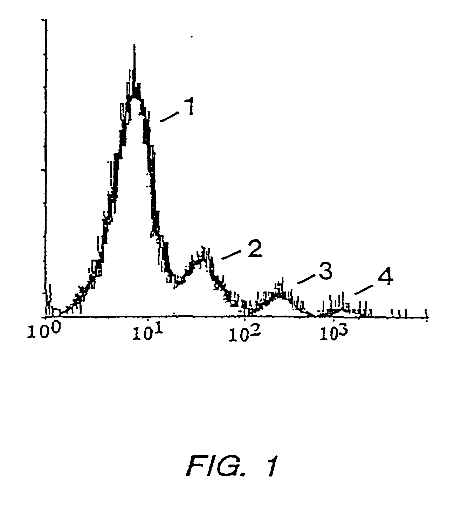

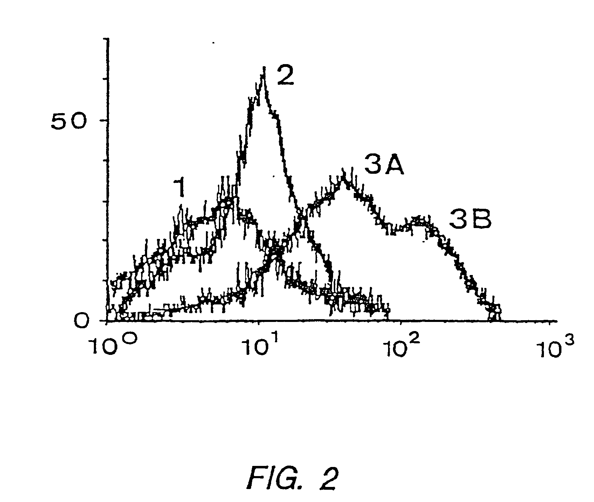

Les figures 1 et 2 montrent le suivi au cours du temps de l'internalisation des macrophages au cytofluorimètre en flux. Sur l'histogramme de la figure 1, on observe 4 pics correspondant à l'internalisation de 1 à 4 érythrocytes par macrophage, après 1 heure de contact entre macrophages et érythrocytes désialylés.

Après deux heures d'incubation, la phagocytose est plus intense : sur l'histogramme de la figure 2 on observe que les 4 pics précédents ont évolué en 2 pics plus intensément marqués : 3A et 3B (intensité moyenne de fluorescence : 112). Les contrôles (fluorescence endogène) sont réalisés avec les macrophages seuls ou les macrophages et érythrocytes en présence d'azide de sodium (pic 1 - intensité moyenne de fluorescence : 59,10) et avec les macrophages incubés en présence d'érythrocytes natifs (pic 2 - intensité moyenne de fluorescence : 67) donc comprenant un faible pourcentage d'érythrocytes sénescents et physiologiquement désialylés.

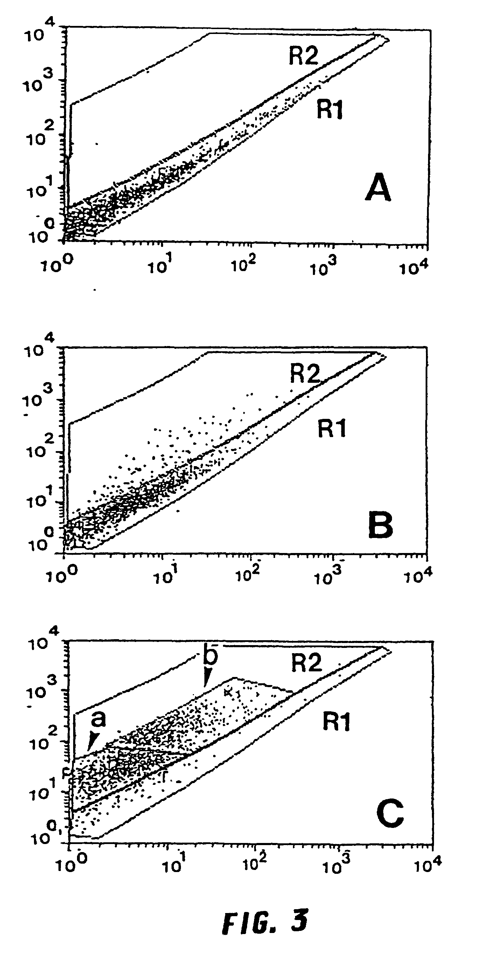

La figure 3 représente une analyse graphique "dot plot" des populations de macrophages phagocytant ou non les érythrocytes, après 2 heures d'incubation.

La région R1 comprend les macrophages seuls, en absence d'érythrocytes et correspond au bruit de fond d'autofluorescence (figure A)

La région R2 comprend les macrophages ayant phagocyté 1 ou plusieurs érythrocytes.

La figure B montre la phagocytose de la population des érythrocytes sénescents naturellement présents dans le sang circulant.

La figure C montre l'augmentation de phagocytose après traitement de désialylation des érythrocytes et fait apparaître 2 sous-populations R2a et R2b dans la zone R2.

Les pourcentages respectifs de distribution des cellules calculés à partir des figures 3 (B et C) sont repris dans le tableau suivant :

| | Capacité de phagocytose de macrophages incubés avec | | | |
|---|---|---|---|---|
| Régions | des érythrocytes natifs | | des érythrocytes désialylés | |
| | % du total | fluorescence moyenne | % du total | fluorescence moyenne |
| R1 | 89.10 | 64,00 | 7,90 | 73,6 |
| R2 | 3,70 | 132,90 | 81,40 | 115,00 |
| R2a | 2,90 | 128,80 | 57,50 | 107,20 |
| R2b | 0,50 | 150,40 | 24,00 | 135,60 |

On voit que 7,9 % des macrophages n'ont pas phagocyté tandis que le pourcentage de macrophages qui phagocytent passe de 3,7 % avec des érythrocytes natifs à 81,4 % avec des érythrocytes désialylés.

Toutes les valeurs expérimentales seront donc comprises entre ces 2 valeurs de référence.

## Revendications

1. Procédé d'évaluation, en routine, de l'état physiologique d'érythrocytes par quantification, par cytofluorimétrie en flux, de la fluorescense de la membrane des érythrocytes marqués par un fluorochrome non toxique (a) mis en contact avec des macrophages en culture pendant le temps nécessaire pour permettre leur phagocytose *in vitro* (b), **caractérisé en ce qu'**il comprend :
c)- une série de lavages pour éliminer les érythrocytes non phagocytés puis la lyse des érythrocytes adhérents, non phagocytés, à la surface externe des macrophages et l'élimination du fluorochrome des membranes résiduelles desdits érythrocytes adhérents par un traitement à l'éthanol à 70 %,
d)- la récolte des macrophages ainsi traités, et
e)- la mesure et l'enregistrement de leur fluorescence.

2. Procédé selon la revendication 1, **caractérisé en ce que** les macrophages sont des macrophages péritonéaux de souris fraîchement prélevés et mis en culture pendant environ 4 heures à 37°C.

3. Procédé selon la revendication 1, **caractérisé en ce que** le fluorochrome est la molécule connue sous l'appellation PKH-26.

4. Procédé selon la revendication 1, **caractérisé en ce que**, à l'étape b), les érythrocytes sont mis en contact avec les macrophages pendant 1 à 6 heures à 37°C.

5. Procédé selon la revendication 1, **caractérisé en ce que**, à l'étape c), la lyse des érythrocytes adhérant à la surface externe des macrophages est réalisée, après 3 lavages en milieu physiologique, par un traitement de 5 minutes en tampon hypotonique.

6. Procédé selon la revendication 1 ou 5, **caractérisé en ce que**, à l'étape c), la culture de macrophages est traitée avec une solution de lidocaïne, puis mise en suspension en tampon physiologique, traitée à l'éthanol à 70 % pendant 20 minutes et remise en suspension en milieu physiologique.

7. Procédé selon la revendication 1, **caractérisé en ce que**, à l'étape e), la mesure du marquage des macrophages récoltés est effectuée par cytofluorimétrie en flux, à deux longueurs d'onde pour distinguer l'autofluorescence des macrophages et la fluorescence de la PKH-26, et que son enregistrement permet de visualiser, sur des histogrammes, deux régions, R1 correspondant à la non-phagocytose et R2 correspondant à la phagocytose, ainsi que des sous-populations de macrophages présentant différents niveaux de phagocytose, R2a et R2b.

8. Utilisation du procédé selon l'une quelconque des revendications 1 à 7 pour évaluer la sénescence des érythrocytes au cours de leur conservation en vue des transfusions sanguines et des tests d'analyses.

9. Utilisation selon la revendication 8 pour évaluer les améliorations des conditions de conservation des érythrocytes.

10. Utilisation du procédé selon l'une quelconque des revendications 1 à 7 pour évaluer la sénescence des érythrocytes dans un prélèvement sanguin pour diagnostiquer des états d'anémie nécessitant une transfusion sanguine.

## Patentansprüche

1. Verfahren zur routinemäßigen Bewertung des physiologischen Zustands von Erythrozyten durch Quantifizierung, mittels Durchflusszytofluorometrie, der Fluoreszenz der Membran von mit einem nicht-toxischen Fluorochrom markierten Erythrozyten (a), die mit Makrophagen in Kultur während eines Zeitraums in Kontakt gebracht werden, der erforderlich ist, um ihre Phagozytose in vitro zu ermöglichen (b), **dadurch gekennzeichnet, dass** es umfasst:
c) eine Reihe von Waschungen, um die nicht phagozytierten Erythrozyten zu entfernen, danach die Lyse von an der äußeren Oberfläche der Makrophagen anhaftenden, nicht phagozytierten Erythrozyten und das Entfemen des Fluorochroms von den übrigbleibenden Membranen der besagten anhaftenden Erythrozyten durch eine Behandlung mit 70 %igem Ethanol,
d) die Ernte der so behandelten Makrophagen, und
e) die Messung und Aufzeichnung ihrer Fluoreszenz.

2. Verfahren nach Anspruch 1, **dadurch gekennzeichnet, dass** die Makrophagen frisch entnommene und ungefähr 4 Stunden bei 37°C kultivierte peritoneale Makrophagen der Maus sind.

3. Verfahren nach Anspruch 1, **dadurch gekennzeichnet, dass** das Fluorochrom das unter der Bezeichnung PKH-26 bekannte Molekül ist.

4. Verfahren nach Anspruch 1, **dadurch gekennzeichnet, dass** im Schritt b) die Erythrozyten 1 bis 6 Stunden bei 37°C mit den Makrophagen in Kontakt gebracht werden.

5. Verfahren nach Anspruch 1, **dadurch gekennzeichnet, dass** im Schritt c) die Lyse der an der äußeren Oberfläche der Makrophagen anhaftenden Erythrozyten nach 3 Waschungen in physiologischem Medium durch eine Behandlung von 5 Minuten in hypotonem Puffer erfolgt.

6. Verfahren nach Anspruch 1 oder 5, **dadurch gekennzeichnet, dass** im Schritt c) die Makrophagenkultur mit einer Lösung von Lidocain behandelt wird, anschließend in physiologischem Puffer in Suspension gebracht wird, mit 70 %igem Alkohol 20 Minuten lang behandelt wird und in physiologischem Medium wieder in Suspension gebracht wird.

7. Verfahren nach Anspruch 1, **dadurch gekennzeichnet, dass** im Schritt e) die Messung der Markierung der geemteten Makrophagen mittels Durchflusszytofluorometrie bei zwei Wellenlängen erfolgt, um die Autofluoreszenz der Makrophagen und die Fluoreszenz des PKH-26 zu unterscheiden, und dass ihre Aufzeichnung ermöglicht, an Histogrammen zwei Regionen, R1, die der Nichtphagozytose entspricht, und R2, die der Phagozytose entspricht, sowie Unterpopulationen von Makrophagen, die verschiedene Niveaus der Phagozytose aufweisen, R2a und R2b, sichtbar zu machen.

8. Verwendung des Verfahrens nach einem der Ansprüche 1 bis 7 zum Bewerten der Alterung von Erythrozyten im Laufe ihrer Aufbewahrung im Hinblick auf Bluttransfusionen und Analysentests.

9. Verwendung nach Anspruch 8 zum Bewerten der Verbesserungen der Aufbewahrungsbedingungen von Erythrozyten.

10. Verwendung des Verfahrens nach einem der Ansprüche 1 bis 7 zum Bewerten der Alterung von Erythrozyten in einer Blutprobe zum Diagnostizieren von Anämie-Zuständen, die eine Bluttransfusion erforderlich machen.

## Claims

1. A method of routinely evaluating the physiological state of erythrocytes by quantifying, by flow cytometry, the fluorescence of the membrane of the erythrocytes labelled with a non-toxic fluorochrome (a) brought into contact with macrophages in culture for the amount of time required to allow them to be phagocytosed *in vitro* (b), **characterized in that** it comprises:
c) - a series of washes in order to eliminate the non-phagocytosed erythrocytes, then lysis of the adherent, non-phagocytosed erythrocytes at the outer surface of the macrophages and elimination of the fluorochome of the residual membranes of said adherent erythrocytes by treatment with 70% ethanol,
d) - harvesting of the macrophages thus treated, and
e) - measurement and recording of their fluorescence.

2. A method according to claim 1, **characterized in that** the macrophages are mouse peritoneal macrophages that have been freshly removed and placed in culture for approximately 4 hours at 37°C.

3. A method according to claim 1, **characterized in that** the fluorochrome is the molecule known under the name PKH-26.

4. A method according to claim 1, **characterized in that**, in step b), the erythrocytes are brought into contact with the macrophages for 1 to 6 hours at 37°C.

5. A method according to claim 1, **characterized in that**, in step c), the lysis of the adherent erythrocytes at the outer surface of the macrophages is carried out, after 3 washes in physiological medium, by means of a treatment for 5 minutes in hypotonic buffer.

6. A method according to claim 1 or 5, **characterized in that**, in step c), the macrophage culture is treated with a solution of lidocaine, and then suspended in physiological buffer, treated with 70% ethanol for 20 minutes and resuspended in physiological medium.

7. A method according to claim 1, **characterized in that**, in step e), the measurement of the labelling of the harvested macrophages is carried out by flow cytofluorometry at two wavelengths so as to distinguish the autofluorescence of the macrophages and the fluorescence of the PKH-26, and **in that** the recording thereof makes it possible to visualize, on histograms, two regions, R1 corresponding to the non-phagocytosis and R2 corresponding to the phagocytosis, and also subpopulations of macrophages exhibiting various levels of phagocytosis, R2a and R2b.

8. The use of the method according to any one of claims 1. to 7, for evaluating the senescence of erythrocytes in the course of their storage with a view to blood transfusions and analytical tests.

9. The use according to claim 8, for evaluating improvements in the conditions for storage of erythrocytes.

10. The use of the method according to any one of claims 1 to 7, for evaluating the senescence of erythrocytes in a blood sample in order to diagnose states of anaemia requiring a blood transfusion.
